# EUROPÄISCHE PATENTSCHRIFT

(11) **EP 0 749 431 B1**
(45) Veröffentlichungstag und Bekanntmachung des Hinweises auf die Patenterteilung: **17.09.1997**
(21) Anmeldenummer: 95911255.8
(22) Anmeldetag: 23.02.1995
(51) Int. Cl.: C07D 301/00

(54) **VERFAHREN ZUR HERSTELLUNG VON 1,2-BUTYLENOXID**
PROCESS FOR PREPARING 1,2-BUTYLENE OXIDE
PROCEDE DE FABRICATION D'OXYDE DE BUTYLENE 1,2

(30) Priorität: 07.03.1994 DE 4407486
(43) Veröffentlichungstag der Anmeldung: 27.12.1996
(73) Patentinhaber: BASF AKTIENGESELLSCHAFT, 67056 Ludwigshafen (DE)
(72) Erfinder: SIGWART, Christoph, D-69198 Schriesheim (DE); BRÖCKER, Franz, Josef, D-67061 Ludwigshafen (DE); FISCHER, Rolf, D-69121 Heidelberg (DE); LINGELBACH, Peter, D-67112 Mutterstadt (DE)
(86) Internationale Anmeldenummer: EP9500656
(87) Internationale Veröffentlichungsnummer: WO9524401

(56) Entgegenhaltungen:
- EP-A- 0 412 515
- EP-A- 0 564 830
- US-A- 4 772 578
- US-A- 5 077 418
- US-A- 5 117 013

## Beschreibung

Die vorliegende Erfindung betrifft ein Verfahren zur Herstellung von 1,2-Butylenoxid durch die katalytische Hydrierung von Vinyloxiran an einem Heterogenkatalysator.

Die katalytische Hydrierung von Vinyloxiran ist bekannt.

Nach US-A 2 561 984 entsteht bei der Hydrierung von Vinyloxiran in Ethanol an einem Palladium-Aktivkohle-Katalysator bei 25°C/2 bar nach einer Reaktionszeit von 3 h als Hauptprodukt n-Butyraldehyd. Mit Raney-Nickel als Katalysator wird dagegen bei 25°C und 2 bar nach einer Reaktionszeit von 1,5 h hauptsächlich n-Butanol gebildet. Über die Bildung von Butylenoxid wird nicht berichtet.

In der Arbeit von Aizikovich et al. (J. Gen. Chem. USSR, 28, 3076 (1958)) wird die katalytische Hydrierung von Vinyloxiran in Methanol bzw. Ethanol an Platin-, Palladium- und Raney-Nickel-Katalysatoren beschrieben. Mit einem Palladium-Trägerkatalysator (1,8 Gew.-% Palladium auf Calziumcarbonat) wird bei 15°C/1 bar hauptsächlich n-Butanol gebildet. Als wichtigste Zwischenverbindung der Hydrierung wird in dieser Schrift Crotylalkohol angesehen, obwohl auch die Bildung von n-Butyraldehyd beobachtet wird. zur Bildung von Butylenoxid gibt es auch in dieser Arbeit keine Hinweise.

In US-A 5 077 418 und US-A 5 117 013 wird berichtet, daß bei der Hydrierung von Vinyloxiran-Lösungen an Palladium-haltigen Katalysatoren als Hauptprodukt n-Butyraldehyd gebildet wird. So erhält man bei der Hydrierung von Vinyloxiran mit Tetrahydrofuran als Lösungsmittel an einem Palladium-Aktivkohle-Trägerkatalysator (5 Gew.-% Palladium auf Aktivkohle) bei einer Temperatur von 50 bis 55°C und einem Druck von 3,5 bar nach einer Reaktionszeit von 3 h einen Hydrieraustrag, der 55 % n-Butyraldehyd, nur 27 % Butylenoxid und 9 % n-Butanol enthält.

Wird die Hydrierung an Palladium-haltigen Aluminiumoxid-Trägerkatalysatoren (5 % Pd/Al₂O₃) durchgeführt, so werden bei einer Temperatur von 25 bis 55°C und einem Druck von 3,5 bar nach einer Reaktionszeit von 6 h bzw. bei einer Temperatur von 100°C und einem Druck von 20,7 bar nach einer Reaktionszeit von 4 h sogar nur Spuren von Butylenoxid gebildet. Bei quantitativem Umsatz entsteht als Hauptprodukt n-Butyraldehyd mit einer Selektivität von 87 % bzw. 78 %.

Außerdem wird die Hydrierung von Vinyloxiran an Raney-Nickel als Hydrierkatalysator bei 50°C und 3,5 bar beschrieben, wobei als Hauptprodukt n-Butanol zu 58 % gebildet wird. Die Butylenoxidausbeute ist mit 41 % niedrig. Bei der Hydrierung von Vinyloxiran an einem Platin-haltigen Trägerkatalysator (1 Gew.-% Pt/Al₂O₃) bei 100°C und 20,7 bar Wasserstoffdruck werden nach einer Reaktionszeit von 4,6 h bei vollständigem Umsatz nur 40 % Butylenoxid neben 23 % n-Butanol, 24 % an verschiedenen Butenolen, 5 % Crotonaldehyd und 3 % n-Butyraldehyd gefunden. Andere Platin-haltige Katalysatoren liefern gar noch geringere Butylenoxid-Ausbeuten.

US-A 5 077 418 und US-A 5 117 013 beschreiben weiterhin, daß hohe Butylenoxid-Ausbeuten nur mit Rhodium-haltigen Katalysatoren erhalten werden. Mit verschiedenen Rhodium-haltigen Trägerkatalysatoren (5 Gew.-% Rhodium auf Aktivkohle; 5 Gew.-% Rhodium auf Aluminiumoxid), die allerdings einen hohen Gehalt an dem teuren Edelmetall Rhodium haben, bzw. mit Rhodiumoxidhydrat (Rh₂O₃ x H₂O) werden bei der Hydrierung von Vinyloxiran-Lösungen Butylenoxid-Gehalte von 60 bis 93 % erhalten. Nachteilig bei diesen Verfahren ist die geringe Raum-Zeit-Ausbeute, bezogen auf die eingesetzte Rhodiummenge. So beträgt die Raum-Zeit-Ausbeute in Beispiel 2 von US-A 5 117 013 nur 119 kg 1,2-Butylenoxid/kg Rh·h.

In Neftekhimiya 33, 131 (1993) wird die Hydrierung von Vinyloxiran an Nickel-, Palladium- und Kupfer-haltigen Katalysatoren beschrieben. Mit Raney-Nickel bzw. Nickel auf Kieselgur als Katalysator verläuft die Hydrierung hauptsächlich unter Spaltung des Epoxid-Rings, die zur überwiegenden Bildung von 1-Butenolen und n-Butanol führt. Die Ausbeuten an Butylenoxid sind gering. Beispielsweise wird an Raney-Nickel mit Methanol als Lösungsmittel bei 40°C/60 bar Wasserstoffdruck nach 20 min Reaktionszeit bei 94 % Umsatz ein Reaktionsaustrag erhalten, der, bezogen auf umgesetztes Vinyloxiran, 89 % Butenole, 8 % n-Butanol und nur 2 % Butylenoxid enthält. Auch mit frisch hergestelltem Raney-Nickel liefert die Hydrierung von Vinyloxiran in Methanol bei 20°C/60 bar Wasserstoffdruck nach 3 min Reaktionszeit bei einem Umsatz von 94 % neben 79 % n-Butanol und 6 % Butenol nur 9 % Butylenoxid. Bei einem Hydrierversuch in Methanol bei 20°C/60 bar Wasserstoffdruck an einem mit einem Gemisch aus Isopropanol, Nikotinsäure, Pyridin und Morpholin vorbehandelten Raney-Nickel-Katalysator wird bei 89 % Umsatz mit 37 % die höchste, mit einem Nickel-haltigen Katalysator erzielbare Butylenoxid-Selektivität erreicht.

Dabei fallen Butenole bzw. n-Butanol in einer Selektivität von 56 % bzw. 9 % an.

Mit Palladium-haltigen Katalysatoren werden bei der Hydrierung von Vinyloxiran im Vergleich zu den Versuchen mit Nickel-haltigen Katalysatoren höhere Butylenoxid-Selektivitäten erzielt. Beispielsweise wird mit einem Palladium/Aktivkohle-Katalysator ohne Einsatz eines Lösungsmittels bei 15°C/60 bar Wasserstoffdruck nach 13 min Reaktionszeit bei 61 %igem Umsatz 81 % Butylenoxid, bezogen auf umgesetztes Vinyloxiran, erhalten. Mit Methanol als Lösungsmittel wird dagegen unter den gleichen Reaktionsbedingungen bei einem Umsatz von 86 % eine Butylenoxid-Selektivität von nur 53 % erhalten, wobei 13 % Butanol und 18 % Butenole gebildet werden. Nachteilig an diesem Verfahren ist, daß eine hohe Selektivität für die Bildung von 1,2-Butylenoxid nur bei einem relativ geringen Teilumsatz des Vinyloxirans erzielt werden. Da Vinyloxiran und 1,2-Butylenoxid destillativ praktisch nicht voneinander getrennt werden können, kommt diesem Verfahren somit keine technische Bedeutung zu. Mit Palladium-Katalysatoren auf Polymerbasis werden bei einem Umsatz von nur 68 % maximale Butylenoxid-Selektivitäten von 60 % erzielt, wobei Butenol bzw. n-Butanol in einer Selektivität von 18 bzw. 4 % gebildet werden.

Mit Kupfer-haltigen Katalysatoren wird eine niedrigere Hydrieraktivität und die Verharzung des Hydrieraustrages beobachtet, die dieses Verfahren technisch impraktikabel macht. Bei Reaktionstemperaturen von 60 bis 100°C, 60 bar Wasserstoffdruck und 30 Gew.-% Katalysator wird nach 3 h Reaktionszeit ein Vinyloxiranumsatz von 50 % und eine Butylenoxid-Selektivität von 70 % erzielt.

Der vorliegenden Erfindung lag somit die Aufgabe zugrunde, ein wirtschaftliches Verfahren zur Herstellung von 1,2-Butylenoxid aus Vinyloxiran zu finden, bei dem 1,2-Butylenoxid in hoher Ausbeute und Selektivität gebildet wird. Weiterhin war es das ziel, für diesen Zweck Katalysatoren zu finden, die diese Aufgabe erfüllen und im Vergleich zu den Katalysatoren des Standes der Technik wesentlich geringere Mengen an teuren Edelmetallen, insbesondere Rhodium oder Palladium, als Katalysatorkomponenente benötigen.

Dementsprechend wurde ein Verfahren zur Herstellung von 1,2-Butylenoxid durch die katalytische Hydrierung von Vinyloxiran an einem Heterogenkatalysator gefunden, das dadurch gekennzeichnet ist, daß man einen Katalysator verwendet, der ein Element aus der I., VII. oder VIII. Nebengruppe des Periodensystems der Elemente oder Gemische dieser Elemente in An- oder Abwesenheit eines oder mehrerer anderer, als Promotoren dienender Elemente enthält, wobei diese Elemente und Promotoren mittels einer Vakuumaufdampftechnik auf einem Träger aus Metallfolie oder Metalldrahtgewebe aufgebracht worden sind.

Das erfindungsgemäße Verfahren ermöglicht es überraschenderweise die Doppelbindung des Vinyloxirans (I) gemäß Gleichung (1) 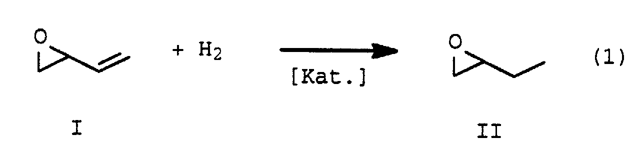 selektiv zu hydrieren, ohne daß der empfindliche Epoxidring bei der Hydrierung in nennenswertem Umfang hydrogenolytisch gespalten wird und ohne daß es in nennenswertem Umfang zu anderen Nebenreaktionen, z.B. Isomerisierungen des Vinyloxirans, beispielsweise zu Butyraldehyd oder Crotonaldehyd, kommt, die in der Folge zu Butanol und Crotylalkohol hydriert werden. Da die erfindungsgemäß angewandten Katalysatoren im Vergleich zu anderen Katalysatoren des Standes der Technik einen sehr geringen Gehalt an den katalytisch aktiven, aber sehr teuren Edelmetallen, wie Rhodium und Palladium, haben, ermöglicht das erfindungsgemäße Verfahren die selektive Herstellung von 1,2-Butylenoxid (II) auf sehr wirtschaftliche Weise.

Die erfindungsgemäß verwendeten Katalysatoren werden hergestellt, indem man mittels einer Vakuumaufdampftechnik die Elemente der I., VII. oder VIII. Nebengruppe des Periodensystems der Elemente, insbesondere Kupfer, Silber, Gold, Rhenium, Ruthenium, Kobalt, Rhodium, Nickel, Palladium oder Platin oder Gemische dieser Elemente auf einen Metallträger aufbringt, der als Metallfolie oder als Metalldrahtgewebe vorliegen kann.

Außer den Elementen der I., VII. oder VIII. Nebengruppe des Periodensystems der Elemente können zusätzlich noch als Promotoren und/oder als Stabilisatoren wirkende Elemente auf den Metallträger aufgebracht werden. Solche Elemente sind z.B. die Elemente der IV. Hauptgruppe und der II. und IV. Nebengruppe des Periodensystems der Elemente, insbesondere die Elemente Blei, Zinn, Silizium, Zink, Cadmium, Titan und Zirkonium. Diese Elemente, in der vorliegenden Anmeldung der Einfachheit halber als Promotoren bezeichnet, können einzeln oder im Gemisch mit anderen Promotorelementen mit einem oder mehreren der Elemente der I., VII. oder VIII. Nebengruppe des Periodensystems der Elemente durch Vakuumverdampfungstechniken auf dem Metallträger abgeschieden werden.

Die katalytisch aktiven Metalle aus der I., VII. oder VIII. Nebengruppe des Periodensystems der Elemente oder Gemische dieser Metalle werden auf dem Metallträger gegebenenfalls zusammen mit einem oder mehreren der Promotorelemente im allgemeinen in einer Menge von im allgemeinen 1 bis 300 mg pro m² Fläche des metallischen Trägermaterials, vorzugsweise in einer Menge von 20 bis 200 mg pro m² Fläche, insbesondere von 40 bis 100 mg pro m² Fläche des metallischen Trägermaterials abgeschieden. Die Promotorelemente aus der IV. Hauptgruppe, der II. oder IV. Nebengruppe des Periodensystems oder Gemische dieser Promotorelemente werden auf dem Metallträger im allgemeinen in einer Menge von jeweils 1 bis 300 mg pro m² Fläche des metallischen Trägermaterials, vorzugsweise in einer Menge von 20 bis 200 mg pro m² Fläche, insbesondere von 40 bis 100 mg pro m² Fläche des metallischen Trägermatarials abgeschieden. Die Abscheidung der einzelnen Katalysatorkomponenten - katalytisch aktive Elemente und Promotorelemente - kann gleichzeitig oder sukzessive erfolgen.

Man kann als Trägermaterialien Metallfolien oder Drahtgewebe aus verschiedenen, webbaren Metalldrähten, wie Eisen, Federstahl, Kupfer, Messing, Phosphorbronze, Reinnickel, Monel, Aluminium, Silber, Neusilber, Nickel, Chromnickel, Chromstahl, Chromnickelstahl sowie Titan verwenden. Als besonders gut geeignete Trägermaterialien haben sich im erfindungsgemäßen Verfahren, Metallfolien und insbesondere Metalldrahtgewebe aus den Werkstoffen mit den Werkstoffnummern 1.4767, 1.4401 und 1.4301, erwiesen. Die Bezeichnung dieser Werkstoffe mit den obengenannten Werkstoffnummern folgt den Angaben der Werkstoffnummern in der "Stahleisenliste", herausgegeben vom Verein Deutscher Eisenhüttenleute; 8. Aufl., S. 87, S. 89 und S. 106, Verlag Stahleisen mbH, Düsseldorf 1990. Der Werkstoff mit der Werkstoffnummer 1.4767 ist auch unter der Bezeichnung Kanthal bekannt.

Als Trägermaterial für die im erfindungsgemäßen Verfahren anwendbaren Katalysatoren können, neben Metallfolien, Metalldrahtgewebe unterschiedlicher Webart, z.B. glatte Gewebe, Köpergewebe, Tressengewebe, Fünfschaft-Atlasgewebe u.a. Spezialbindungsgewebe, verwendet werden. Auch Formkörper aus Metallfolien, Metalldrahtgeweben oder Drahtgestrick, z.B. Spiralen, Raschigringe oder Monolithe, können vorteilhaft als Trägermaterialien eingesetzt werden.

Vorteilhaft werden diese Metallträger vor ihrer Beschichtung mit den katalytisch aktiven Metallen und gegebenenfalls den Promotoren einer oxidativen Temperung unterzogen. Dazu werden diese Träger, vorzugsweise an der Luft auf Temperaturen von 600 bis 1100°C, vorzugsweise auf 750 bis 1000°C, erhitzt.

Die Metallfolie oder das Metalldrahtgewebe wird, gegebenenfalls nach vorausgegangener oxidativer Temperung, mittels einer Vakuumaufdampftechnik mit "dünnen Schichten" der katalytisch aktiven Metalle und gegebenenfalls mit den Promotoren beschichtet. Als "dünne Schichten" werden Beläge mit einer Schichtdicke von wenigen Angström und maximal einigen Mikrometern bezeichnet. Die Schichtdicke kann mittels eines Schwingquarzes gemessen werden.

Als Vakuumaufdampftechniken können verschiedene Verfahren, z.B. die thermische Verdampfung, die Flash-Verdampfung, die Kathodenzerstäubung sowie die Kombination von thermischer Verdampfung und Kathodenzerstäubung eingesetzt werden. Die thermische Verdampfung kann durch direkte oder indirekte elektrische Beheizung erfolgen. Auch die Elektronenstrahl-Verdampfung kann eingesetzt werden. Dabei wird die zu verdampfende Substanz in einem wassergekühlten Tiegel mit einem Elektronenstrahl oberflächlich so stark erhitzt, daß selbst hochschmelzende Metalle und Dielektrika verdampft werden können.

Mit diesen Methoden kann man den Katalysatorträger gezielt mit den katalytisch aktiven Elementen und gegebenenfalls den Promotoren in optimaler Weise beschichten. Legierungen werden vorzugsweise durch Flash-Verdampfung aufgebracht.

Der Metallträger kann in einer Vakuumbedampfungsanlage diskontinuierlich oder kontinuierlich bedampft werden, z.B. indem die aufzubringende Aktivkomponente, im Vakuum bei 10⁻² bis 10⁻¹⁰, vorzugsweise 10⁻⁴ bis 10⁻⁸ Torr, mittels eines Elektronenstrahls so stark erhitzt wird, daß das Metall aus dem wassergekühlten Tiegel heraus verdampft und sich auf dem Träger niederschlägt. Das Trägermaterial wird zweckmäßigerweise so angeordnet, daß ein möglichst großer Teil des Dampfstroms auf dem Träger kondensiert. Durch eine eingebaute Wickelmaschine können die Metallträger kontinuierlich beschichtet werden. Drahtgestricke, wie z.B. Raschigringe aus feinen Drähten, können z.B. in einem Drehtrommelkorb für Schüttgut gefüllt und darin bedampft werden.

Die zur Herstellung der erfindungsgemäß verwendbaren Katalysatoren anwendbaren Vakuumaufdampftechniken sind selbst nicht Gegenstand der vorliegenden Erfindung. Bezüglich der Einzelheiten zur Durchführung der Beschichtung der Metallträger mit katalytisch aktiven Metalle und Promotoren sei deshalb auf Handbücher verwiesen z.B. Maissel, Glang "Handbook of Thin Film Technology", McGraw Hill, New York, 1970 und Vossen, Kern "Thin Film Processes", Academic Press, New York, 1978 oder US-A 4 686 202.

Nach vollzogener Beschichtung kann es sich als vorteilhaft erweisen, das beschichtete Trägermaterial noch einer thermischen Konditionierung zu unterziehen, wobei es, vorzugsweise in Gegenwart von Luft oder Sauerstoff bei Temperaturen von im allgemeinen 20 bis 800°C, vorzugsweise von 25 bis 700°C, während einer Zeitdauer von im allgemeinen 0,5 bis 2 h behandelt wird. Falls nicht schon bei der Beschichtung des Metallträgers Formkörper aus Metallfolien oder Metalldrahtgeweben eingesetzt werden, können die Metallfolien oder Metalldrahtgewebe auch nach der Beschichtung und Konditionierung gewünschtenfalls zu Formkörpern, wie Raschigringen, Spiralen oder Monolithen, verarbeitet werden.

Die so hergestellten Katalysatoren können direkt im erfindungsgemäßen Verfahren verwendet werden, vorteilhaft werden sie vor ihrem Einsatz im erfindungsgemäßen Verfahren reduziert, und zwar im allgemeinen mit Wasserstoff oder Wasserstoff-haltigen Gasen bei Temperaturen von typischerweise 50 bis 300°C; vorzugsweise von 80 bis 250°C. Die Reduktion wird im allgemeinen solange durchgeführt, bis kein Wasser mehr gebildet wird.

Zur Durchführung des erfindungsgemäßen Verfahrens wird das Vinyloxiran oder Lösungen des Vinyloxirans in einem unter den Reaktionsbedingungen inerten Lösungsmittel in Gegenwart der erfindungsgemäß zu verwendenden Katalysatoren bei Temperaturen von im allgemeinen 0 bis 200°C, vorzugsweise von 10 bis 130°C, insbesondere von 20 bis 100°C und besonders bevorzugt von 40 bis 100°C bei einem Druck von im allgemeinen 1 bis 300 bar, vorzugsweise von 1 bis 100 bar und besonders bevorzugt von 1 bis 50 bar hydriert.

Das erfindungsgemäße Verfahren kann ohne Lösungsmittel oder vorteilhaft in Anwesenheit eines unter den Reaktionsbedingungen inerten Lösungsmittels durchgeführt werden. Derartige Lösungsmittel können beispielsweise sein: Ether, wie Tetrahydrofuran, Dioxan, Methyl-tert.-butylether, Di-n-butylether, Dimethoxyethan oder Diisopropylether, Alkohole, wie Methanol, Ethanol, Propanol, Isopropanol, n-Butanol, Isobutanol oder tert.-Butanol, C₂- bis C₄-Glykole, Kohlenwasserstoffe, wie Petrolether, Benzol, Toluol oder Xylol, N-Alkyllactame, wie N-Methylpyrrolidon oder N-Octylpyrrolidon.

Das erfindungsgemäße Verfahren kann sowohl kontinuierlich als auch diskontinuierlich, in der Gasphase oder in der flüssigen Phase ausgeübt werden. Bei der kontinuierlichen Betriebsweise können beispielsweise Rohrreaktoren vorteilhaft eingesetzt werden, in denen der Katalysator vorzugsweise in Form von Monolithen angeordnet ist, über die die Reaktionsmischung in Sumpf- oder Rieselfahrweise geleitet werden kann. Bei der diskontinuierlichen Betriebsweise können sowohl einfache Rührreaktoren oder vorteilhaft Schlaufenreaktoren verwendet werden. Bei Benutzung von Schlaufenreaktoren wird der Katalysator zweckmäßigerweise in Form von Monolithen oder Raschigringen im Reaktor angeordnet.

Die Aufarbeitung der Reaktionsmischung zur Isolierung des 1,2-Butylenoxids kann auf herkömmliche Weise, z.B. durch Destillation, erfolgen.

Das als Ausgangsmaterial benötigte Vinyloxiran kann z.B. nach dem Verfahren von US-A 4 897 498 durch partielle Oxidation von 1,3-Butadien an Silberkatalysatoren hergestellt werden.

1,2-Butylenoxid findet z.B. als Kraftstoffadditiv oder als Stabilisator von Chlorkohlenwasserstoffen Verwendung.

### Beispiele

In sämtlichen Beispielen wurde als Vakuumbedampfungsanlage eine handelsübliche Elektronenstrahlbedampfungsanlage der Fa. Balzers AG, Balzers (Fürstentum Liechtenstein) verwendet.

### Beispiel 1

Ein Drahtgewebe in Leinwandbindung aus dem Werkstoff mit der Nr. 1.4767 mit einer Maschenweite von 0,18 mm und einem Drahtdurchmesser von 0,112 mm wurde 5 h bei 950°C an der Luft erhitzt. Anschließend wurde das so vorbehandelte Trägergewebe in einer Elektronenstrahlbedampfungsanlage mit Palladium bedampft. Die niedergeschlagene Palladium-Menge betrug 92 mg Palladium pro m² Fläche des Gewebes. Aus dem so hergestellten Katalysatorgewebe wurden nach dessen Konditionierung an der Luft bei 25°C 5 x 5 mm große Raschigringe hergestellt. In einem 50 ml fassenden Autoklaven wurde die zu hydrierende Lösung aus 2,5 g Vinyloxiran und 22,5 g Tetrahydrofuran mit 20 cm² Palladium-beschichtetem Katalysatorgewebe in Form von Raschigringen versetzt und unter Rühren 6 h bei 50°C und 40 bar mit Wasserstoff hydriert. Die eingesetzte Menge an Palladium betrug somit 184 µg. Bei einem Umsatz von 100 % wurden 78,4 mol-% Butylenoxid, 2,7 mol-% n-Butyraldehyd und 3,3 mol-% n-Butanol erhalten.

### Beispiel 2

Das gleiche Drahtgewebe wie in Beispiel 1 wurde 5 h bei 1000°C an der Luft geglüht. Nach Abkühlung wurde dieses Trägermaterial in der gleichen Vakuumbedampfungsanlage wie in Beispiel 1 nacheinander mit 92 mg Palladium pro m² Fläche des Trägers und 21,3 mg Blei pro m² Trägerfläche beschichtet. Zur Konditionierung des Katalysators wurde das bedampfte Gewebe an der Luft 0,5 h bei 600°C getempert. Anschließend wurde das Gewebe zu 5 x 5 mm Raschigringen verformt, die in einem Quarzrohr bei 200°C bei Atmosphärendruck mit Wasserstoff aktiviert und unter einer Stickstoff-Atmosphäre abgekühlt wurden. In dieser Form wurden 15 cm² des Katalysators für die Hydrierung von 2,5 g Vinyloxiran in 22,5 g Tetrahydrofuran eingesetzt. Die Hydrierung erfolgte wie in Beispiel 1 beschrieben. Bei einem Umsatz von 100 % wurden 83,9 mol-% 1,2-Butylenoxid, 1,9 mol-% n-Butyraldehyd und 2,4 mol-% n-Butanol erhalten.

### Beispiel 3

Wie in Beispiel 1 beschrieben wurde das gleiche Drahtgewebe 5 h bei 1000°C an der Luft erhitzt und anschließend im Vakuum nacheinander mit 92 mg Palladium pro m² Trägerfläche und 20,2 mg Gold pro m² Trägerfläche bedampft und konditioniert. Nach Herstellung von 5 x 5 mm Raschigringen wurden 18,75 cm² dieses Katalysators in einen Autoklaven gegeben und die Hydrierung von 2,5 g Vinyloxiran in 22,5 g Tetrahydrofuran 8 h bei 50°C und 40 bar Wasserstoffdruck durchgeführt. Bei einem Umsatz von 100 % wurden 89,1 mol-% 1,2-Butylenoxid, 1,7 mol-% n-Butyraldehyd und 1,0 mol-% n-Butanol erhalten.

### Beispiel 4

Das gleiche Drahtgewebe wie in den Beispielen 1 bis 3 wurde 5 h bei 950°C geglüht und nach Abkühlung im Vakuum mit 46 mg Palladium pro m² Fläche und 6,1 mg Silicium pro m² Fläche beschichtet und konditioniert. Nach dem Verformen des Katalysatorgewebes zu 5 x 5 mm Raschigringen wurden 20 cm² dieses Katalysators für den Hydrierversuch eingesetzt, der unter gleichen Reaktionsbedingungen, wie in Beispiel 3 beschrieben, durchgeführt wurde. Bei einem Umsatz von 97,7 % wurden 85,3 mol-% 1,2-Butylenoxid, 1,2 mol-% n-Butyraldehyd und 1,3 mol-% n-Butanol erhalten.

### Beispiel 5

Das Drahtgewebe gemäß den Beispielen 1 bis 4 wurde 5 h bei 900°C geglüht und anschließend in der Vakuumbedampfungsanlage nacheinander mit 46 mg Palladium pro m² Fläche und 19,7 mg Zirkonium pro m² Fläche bedampft und wie in Beispiel 1 konditioniert. Aus dem Katalysatorgewebe wurden 5 x 5 mm Raschigringe geformt und in dieser Form 20,0 cm² des Katalysators in den Hydrierautoklaven gegeben. Anschließend wurden 2,5 g Vinyloxiran in 22,5 g Tetrahydrofuran 4 h bei 50°C und 40 bar Wasserstoffdruck hydriert. Bei quantitativem Umsatz wurden 84,6 mol-% 1,2-Butylenoxid, 1,6 mol-% n-Butyraldehyd und 1,3 mol-% n-Butanol erhalten.

### Beispiel 6

Das gleiche Drahtgewebe wie in den Beispielen 1 bis 5 wurde nach der gleichen Vorbehandlung wie in Beispiel 5 im Vakuum nacheinander mit 46 mg Palladium pro m² Fläche und 10,4 mg Titan pro m² Fläche beschichtet und wie in Beispiel 1 konditioniert. Das so erhaltene Katalysatorgewebe wurde zu 5 x 5 mm Raschigringen verformt und davon 21,25 cm² für den Autoklavenversuch verwendet. Dieser wurde unter den gleichen Reaktionsbedingungen, wie in Beispiel 5 beschrieben, durchgeführt. Bei vollständigem Umsatz wurden 82,4 mol-% Butylenoxid, 1,6 mol-% n-Butyraldehyd und 2,1 mol-% n-Butanol erhalten.

### Beispiel 7

Das gleiche Drahtgewebe wie in den Beispielen 1 bis 6 wurde 5 h bei 1000°C an der Luft geglüht. Anschließend wurde in der Vakuumbedampfungsanlage Nickel in einer Menge von 66 mg Nickel pro m² Fläche aufgedampft und wie in Beispiel 1 konditioniert. Aus diesem Katalysatorgewebe wurden 5 x 5 mm Raschigringe hergestellt, die mit Wasserstoff bei 230°C wie in Beispiel 2 beschrieben, aktiviert wurden. 22,5 cm² des aktivierten Katalysators wurden für den Hydrierversuch eingesetzt, der unter den gleichen Reaktionsbedingungen, wie in Beispiel 3 beschrieben, durchgeführt wurde. Bei einem Umsatz von 94,8 % wurden 80,8 mol-% 1,2-Butylenoxid, 1,7 mol-% n-Butyraldehyd und 4,1 mol-% n-Butanol erhalten.

### Beispiel 8

Das gleiche Trägergewebe wie in den Beispielen 1 bis 7 wurde 5 h an der Luft bei 950°C erhitzt. Nach dem Abkühlen wurde es im Vakuum nacheinander mit 66 mg Nickel pro m² Fläche und 92 mg Palladium pro m² Fläche beschichtet und wie in Beispiel 1 konditioniert. Nachdem das Katalysatorgewebe zu 5 x 5 mm Raschigringen verformt worden war, wurde der Katalysator mit Wasserstoff wie in Beispiel 7 beschrieben, aktiviert. Für den Hydrierversuch wurden 21,25 cm² des aktivierten Katalysators eingesetzt. Unter gleichen Reaktionsbedingungen, wie in Beispiel 1 beschrieben, wurden bei vollständigem Umsatz 84,0 mol-% Butylenoxid, 2,3 mol-% n-Butyraldehyd und 3,5 mol-% n-Butanol erhalten.

### Beispiel 9

Das gleiche Gewebe wie in den Beispielen 1 bis 8 wurde an der Luft 5 h bei 950°C erhitzt und nach dem Abkühlen in der Vakuumverdampfungsanlage nacheinander mit 66 mg Nickel pro m² Fläche und 16,1 mg Rhenium pro m² Fläche bedampft. Zur Konditionierung wurde das beschichtete Gewebe innerhalb von 2 h auf 600°C an der Luft aufgeheizt und 0,5 h bei dieser Temperatur getempert. Anschließend wurden 5 x 5 mm Raschigringe aus dem Katalysatorgewebe gefertigt und 21,25 cm² des Katalysatorgewebes für den Hydrierversuch verwendet. Unter gleichen Reaktionsbedingungen, wie in Beispiel 3 beschrieben, wurden bei quantitativem Umsatz 82,6 mol-% 1,2-Butylenoxid, 1,9 mol-% n-Butyraldehyd und 4,3 mol-% n-Butanol erhalten.

### Beispiel 10

Das Gewebe der Beispiele 1 bis 9 wurde 5 h bei 1000°C geglüht und nach Abkühlung im Vakuum nacheinander mit 66 mg Nickel pro m² Fläche, 16,1 mg Palladium pro m² Fläche und 16,1 mg Rhenium pro m² Fläche beschichtet. Anschließend wurde das Gewebe in 2 h auf 600°C an der Luft erhitzt und bei dieser Temperatur 0,5 h getempert. Nach der üblichen Herstellung von 5 x 5 mm Raschigringen erfolgte die Aktivierung des Katalysators bei Atmosphärendruck mit Wasserstoff bei 200°C in einem Quarzrohr. 21,25 cm² des aktivierten Katalysatorgewebes wurden für den Hydrierversuch benutzt, der unter den gleichen Reaktionsbedingungen, wie in Beispiel 3 beschrieben, durchgeführt wurde. Bei einem Umsatz von 97,2 % wurden 82,2 mol-% 1,2-Butylenoxid, 1,7 mol-% n-Butyraldehyd und 4,6 mol-% n-Butanol erhalten.

### Beispiel 11

Das gleiche Gewebe wie in den vorhergehenden Beispielen wurde 5 h bei 1000°C an der Luft erhitzt. Auf das so vorbehandelte Trägermaterial wurden im Vakuum 88 mg Rhodium pro m² Fläche aufgedampft. Anschließend wurde das beschichtete Gewebe wie in Beispiel 1 konditioniert. Aus dem so gewonnenen Katalysatorgewebe wurden 5 x 5 mm Raschigringe gefertigt und für den Hydrierversuch eine Katalysatorgewebemenge von 21,25 cm² eingesetzt. Bei der unter den gleichen Reaktionsbedingungen, wie in Beispiel 3 beschrieben, durchgeführten Hydrierung von Vinyloxiran wurden nach vollständigem Umsatz 86,0 mol-% 1,2-Butylenoxid, 4,0 mol-% n-Butyraldehyd und 4,5 mol-% n-Butanol erhalten. Die Raum-Zeit-Ausbeute, bezogen auf die eingesetzte Rhodiummenge, betrug 1437 kg 1,2-Butylenoxid/kg Rh·h.

### Beispiel 12

Bei 1000°C 5 h an der Luft erhitztes Gewebe gemäß dem vorhergehenden Beispielen wurde in der Elektronenstrahlbedampfungsanlage nacheinander mit 24 mg Rhodium pro m² Fläche und 46 mg Palladium pro m² Fläche bedampft. Zur Konditionierung wurde das so erhaltene Material während 1 h an der Luft auf 500°C erhitzt und bei dieser Temperatur 1 h getempert. Nach Herstellung von 5 x 5 mm Raschigringen aus dem so erhaltenen Katalysatorgewebe wurden 21,25 cm² für den Hydrierversuch eingesetzt. Die Hydrierung von 2,5 g Vinyloxiran und 22,5 g Tetrahydrofuran bei 50°C und 40 bar Wasserstoffdruck ergab nach 2 h bei quantitativem Umsatz 90,1 mol-% 1,2-Butylenoxid, 1,8 mol-% n-Butyraldehyd und 2,5 mol-% n-Butanol.

### Beispiele 13 bis 17

20 cm² des mit Palladium beschichteten Katalysatorgewebes aus Beispiel 1 wurden für Hydrierversuche zur Hydrierung von 2,5 g Vinyloxiran in 22,5 g unterschiedlichen Lösungsmitteln bei 50°C und 40 bar Wasserstoffdruck eingesetzt. Die Zusammensetzungen der Reaktionsausträge sind in der Tabelle angegeben.

**Tabelle**

| Bsp. | Lösungsmittel | Reaktionszeit | Zusammensetzung des Reaktionsaustrags | | | |
|---|---|---|---|---|---|---|
| | | | VO | BO | n-BA | n-BuOH |
| 13 | MTBE | 1 h | - | 83,4 | 2,3 | 1,7 |
| 14 | Di-n-butylether | 2 h | - | 78,9 | 0,9 | 1,0 |
| 15 | Methanol | 2 h | - | 78,8 | 2,6 | 7,2 |
| 16 | Ethanol | 2 h | - | 75,1 | 1,0 | 2,5 |
| 17 | Toluol | 2 h | - | 77,0 | 1,8 | 2,5 |
| VO = Vinyloxiran BO = 1,2-Butylenoxid n-BA = n-Butyraldehyd n-BuOH = n-Butanol MTBE = Methyl-tert.-butylether | | | | | | |

### Beispiel 18

Drahtgewebe in Leinwandbindung aus dem Werkstoff mit der Nr. 1.4301 mit einer Maschenweite von 0,125 mm und einem Drahtdurchmesser von 0,100 mm wurde 3 h bei 800°C an der Luft erhitzt. Anschließend wurde das so hergestellte Trägermaterial in einer Elektronenstrahlbedampfungsanlage mit Palladium beschichtet. Die aufgebrachte Palladiummenge betrug 92 mg Palladium pro m² Fläche Trägergewebe. Aus dem so hergestellten Katalysator wurden 5 x 5 mm große Raschigringe geformt. 2,5 g Vinyloxiran und 22,5 g Tetrahydrofuran wurden in einen 50 ml fassenden Autoklaven gefüllt, mit 21,25 cm² des verformten Katalysatorgewebes versetzt und unter Rühren 7 h bei 50°C und 40 bar Wasserstoffdruck hydriert. Bei einem Umsatz von 97,7 % wurden 81,0 mol-% Butylenoxid, 1,6 mol-% n-Butyraldehyd und 4,6 mol-% n-Butanol bezogen auf umgesetztes Vinyloxiran erhalten.

### Beispiel 19

Drahtgewebe in Leinwandbindung aus dem Werkstoff mit der Werkstoff Nr. 1.4401, Maschenweite 0,160 mm, Drahtdurchmesser 0,100 mm, wurde 3 h bei 850°C an der Luft erhitzt, danach im Vakuum mit 92 mg Palladium pro m² Fläche bedampft und wie in Beispiel 1 konditioniert. Analog Beispiel 18 wurde mit 21,25 cm² dieses Katalysatorgewebes in Form von Raschigringen Vinyloxiran in Tetrahydrofuran hydriert. Bei 99,5 % Umsatz enthielt der Reaktionsaustrag 79,3 mol-% 1,2-Butylenoxid, 1,9 mol-% n-Butyraldehyd sowie 3,4 mol-% n-Butanol.

### Beispiel 20

Drahtgewebe aus dem Werkstoff Nr. 1.4767 wurde, wie in Beispiel 1 beschrieben, vorbehandelt, im Vakuum mit 161,2 mg Rhenium pro m² Fläche Gewebe bedampft und wie in Beispiel 1 konditioniert. Mit 42,5 cm² des so hergestellten Katalysatorgewebes in Form von 5 x 5 mm Raschigringen wurde die Vinyloxiranhydrierung bei 95°C und 40 bar Wasserstoffdruck wie in Beispiel 18 beschrieben durchgeführt. Nach einer Reaktionszeit von 8 h enthielt das Reaktionsgemisch bei 29,3 % Umsatz 76,0 mol-% 1,2-Butylenoxid, 4,4 mol-% n-Butyraldehyd und 4,7 mol-% n-Butanol, bezogen auf umgesetztes Vinyloxiran.

### Beispiel 21

Das Drahtgewebe gemäß Beispiel 1 wurde an der Luft 5 h bei 950°C geglüht, anschließend im Vakuum mit 91,2 mg Ruthenium pro m² Fläche Gewebe bedampft und wie in Beispiel 1 konditioniert. Von diesem Katalysatorgewebe wurden 42,5 cm² in Form von 5 x 5 mm Raschigringen bei der Hydrierung verwendet. Hydriert wurde bei 95°C und einem Wasserstoffdruck von 40 bar. Bei einem Umsatz von 31,3 % wurden nach 4 h Reaktionszeit 83,4 mol-% 1,2-Butylenoxid, 3,1 mol-% n-Butyraldehyd und 2,5 mol-% n-Butanol, bezogen auf umgesetztes Vinyloxiran, erhalten.

### Beispiel 22

Auf das gemäß Beispiel 1 vorbehandelte Katalysatorgewebe wurden 166,4 mg Kobalt pro m² Gewebe aufgedampft und wie in Beispiel 1 konditioniert. 21,25 cm² dieses Katalysatorgewebes wurden in Form von Raschigringen (5 x 5 mm) für die Hydrierung von Vinyloxiran eingesetzt. Es wurde bei 50°C und 40 bar Wasserstoffdruck hydriert. Bei 40 % Umsatz enthielt der Reaktionsaustrag nach einer Reaktionszeit von 24 h 83,2 mol-% 1,2-Butylenoxid, 1,8 mol-% n-Butyraldehyd sowie 2,6 mol-% n-Butanol, bezogen auf umgesetztes Vinyloxiran.

### Beispiel 23

Auf das gemäß Beispiel 1 vorbehandelte Metallgewebe wurden im Vakuum 67 mg Kupfer pro m² Fläche Gewebe aufgedampft und wie in Beispiel 1 konditioniert. Mit 42,5 cm² dieses Katalysatorgewebes in Form von Raschigringen (5 x 5 mm) wurde die Vinyloxiranhydrierung bei 95°C und 40 bar Wasserstoffdruck durchgeführt. Bei 18 % Umsatz wurden nach 8 h Reaktionszeit 89,6 mol-% 1,2-Butylenoxid, bezogen auf umgesetztes Vinyloxiran, gebildet.

### Beispiel 24

Auf das gemäß Beispiel 1 vorbehandelte Metallgewebe wurden 160 mg Platin pro m² Fläche Gewebe aufgedampft und wie in Beispiel 1 konditioniert. Zur Hydrierung von Vinyloxiran wurden 21,25 cm² dieses Katalysators in Form von Raschigringen (5 x 5 mm) benutzt. Die Hydrierung bei 50°C und 40 bar Wasserstoffdruck lieferte bei einem Umsatz von 85,4 % nach einer Reaktionszeit von 24 h 64,4 mol-% 1,2-Butylenoxid, 3,6 mol-% n-Butyraldehyd und 16,4 mol-% n-Butanol, bezogen auf umgesetztes Vinyloxiran.

### Beispiel 25

Das Katalysatorgewebe gemäß Beispiel 5 wurde zu Raschigringen geformt und für den Hydrierversuch in einer Menge von 40,0 cm² eingesetzt. Die Hydrierung von 10,0 g Vinyloxiran in Abwesenheit eines Lösungsmittels bei 50°C und 40 bar Wasserstoffdruck ergab nach 7 h Reaktionszeit bei quantitativem Umsatz 83,9 mol-% 1,2-Butylenoxid, 1,0 mol-% n-Butyraldehyd und 0,8 mol-% n-Butanol. Die Raum-Zeit-Ausbeute, bezogen auf die eingesetzte Palladiummenge, betrug 6522 kg 1,2-Butylenoxid/kg Pd·h.

## Patentansprüche

1. Verfahren zur Herstellung von 1,2-Butylenoxid durch die katalytische Hydrierung von Vinyloxiran an einem Heterogenkatalysator, dadurch gekennzeichnet, daß man einen Katalysator verwendet, der ein Element aus der I., VII. oder VIII. Nebengruppe des Periodensystems der Elemente oder Gemische mehrerer dieser Elemente in An- oder Abwesenheit eines oder mehrerer, als Promotoren dienender Elemente enthält, wobei diese Elemente und Promotoren mittels einer Vakuumaufdampftechnik auf einem Träger aus Metallfolie oder Metalldrahtgewebe aufgebracht worden sind.

2. Verfahren nach Anspruch 1, dadurch gekennzeichnet, daß man einen Katalysator verwendet, der als Promotoren eines oder mehrere Elemente aus der Hauptgruppe IV., der Nebengruppe II. oder IV. des Periodensystems der Elemente enthält.

3. Verfahren nach den Ansprüchen 1 oder 2, dadurch gekennzeichnet, daß man als Träger Metallfolien oder Metalldrahtnetze aus Werkstoffen mit den Werkstoffnummern 1.4767, 1.4401 oder 1.4301 verwendet.

4. Verfahren nach den Ansprüchen 1 bis 3, dadurch gekennzeichnet, daß man vor dem Aufbringen der katalytisch aktiven Elemente den Träger an der Luft bei Temperaturen von 600 bis 1100°C erhitzt.

5. Verfahren nach den Ansprüchen 1 bis 4, dadurch gekennzeichnet, daß man Katalysatoren verwendet, die eines oder mehrere der Elemente aus der I., VII. oder VIII. Nebengruppe des Periodensystems der Elemente in einer Menge von jeweils 1 bis 300 mg/m² Fläche des Trägers auf dem Metallträger aufgebracht enthalten.

6. Verfahren nach den Ansprüchen 1 bis 5, dadurch gekennzeichnet, daß man Katalysatoren verwendet, die zusätzlich zu den Elementen der I., VII. oder VIII. Nebengruppe des Periodensystems der Elemente zusätzlich noch als Promotoren dienende Elemente enthalten, wobei diese Promotorelemente in einer Menge von jeweils 1 bis 300 mg/m² Fläche des Trägers auf dem Metallträger aufgebracht worden sind.

7. Verfahren nach den Ansprüchen 1 bis 6, dadurch gekennzeichnet, daß man einen Katalysator verwendet, der nach Aufbringung der katalytisch aktiven Elemente mittels einer Vakuumaufdampfungstechnik an der Luft bei Temperaturen von 20 bis 800°C konditioniert worden ist.

8. Verfahren nach den Ansprüchen 1 bis 7, dadurch gekennzeichnet, daß man einen Katalysator verwendet, der vor seiner Verwendung mit Wasserstoff bei Temperaturen von 20 bis 300°C reduziert worden ist.

## Claims

1. A process for preparing 1,2-butylene oxide by catalytic hydrogenation of vinyloxirane over a heterogeneous catalyst, which comprises using a catalyst comprising an element of subgroup I, VII or VIII of the periodic table, or mixtures of a plurality of these elements, in the presence or absence of one or more promoter elements, these elements and promoters having been applied by means of a vacuum vapor deposition technique to a support of metal -foil or metal wire fabric.

2. A process as claimed in claim 1, wherein the catalyst used comprises one or more elements of main group IV or of sub' group II or IV of the periodic table as promoters.

3. A process as claimed in claim 1 or 2, wherein the metal foil or metal wire fabric support used are composed of materials bearing the material numbers 1.4767, 1.4401 or 1.4301.

4. A process as claimed in any of claims 1 to 3, wherein the support is heated in air at from 600 to 1100°C before the catalytically active elements are applied.

5. A process as claimed in any of claims 1 to 4, wherein the catalyst used comprises one or more elements of subgroup I, VII or VIII of the periodic table applied to the metal support in an amount of in each case from 1 to 300 mg/m² of area of the support.

6. A process as claimed in any of claims 1 to 5, wherein the catalyst used, as well as the elements of subgroup I, VII or VIII of the periodic table, further comprises promoter elements, these promoter elements having been applied to the metal support in an amount of in each case from 1 to 300 mg/m² of area of the support.

7. A process as claimed in any of claims 1 to 6, wherein the catalyst used has been conditioned in air at from 20 to 800°C after the catalytically active elements were applied by means of a vacuum vapor deposition technique.

8. A process as claimed in any of claims 1 to 7, wherein the catalyst used was reduced with hydrogen at from 20 to 300°C before use.

## Revendications

1. Procédé pour fabriquer de l'oxyde de 1,2-butylène, par l'hydrogénation catalytique du vinyloxiranne sur un catalyseur hétérogène, caractérisé en ce qu'on utilise un catalyseur qui contient un élément des groupes IB, VIIB et VIII du Tableau Périodique des Eléments, ou des mélanges de plusieurs de ces éléments, en présence ou en l'absence d'un ou plusieurs éléments servant de promoteurs, ces éléments et promoteurs ayant été appliqués, par une technique d'évaporation sous vide, sur un support constitué d'une feuille métallique ou d'un tissu de fil métallique.

2. Procédé selon la revendication 1, caractérisé en ce qu'on utilise un catalyseur qui contient en tant que promoteurs un ou plusieurs des éléments des groupes IVA, IIB ou IVB du Tableau Périodique des Eléments.

3. Procédé selon les revendications 1 ou 2, caractérisé en ce qu'on utilise comme support des feuilles métalliques ou des treillis en fil métallique constitués de matériaux portant les numéros de matériau 1.4767, 1.4401 ou 1.4301.

4. Procédé selon les revendications 1 à 3, caractérisé en ce que, avant application des éléments catalytiquement actifs, on chauffe le support à l'air à des températures de 600 à 1100°C.

5. Procédé selon les revendications 1 à 4, caractérisé en ce qu'on utilise des catalyseurs qui contiennent un ou plusieurs des éléments des groupes IB, VIIB ou VIII du Tableau Périodique des Eléments, appliqués sur le support métallique en une quantité, pour chacun d'eux, de 1 à 300 mg/m² d'aire du support.

6. Procédé selon les revendications 1 à 5, caractérisé en ce qu'on utilise des catalyseurs qui contiennent encore des éléments servant de promoteurs, et ce en plus des éléments des groupes IB, VIIB et VIII du Tableau Périodique des Eléments, ces éléments promoteurs ayant été appliqués sur le support métallique en une quantité, pour chacun, de 1 à 300 mg/m² d'aire du support.

7. Procédé selon les revendications 1 à 6, caractérisé en ce qu'on utilise un catalyseur qui, après application des éléments catalytiquement actifs, a été conditionné à l'aide d'une technique d'évaporation sous vide à l'air à des températures de 20 à 800°C.

8. Procédé selon les revendications 1 à 7, caractérisé en ce qu'on utilise un catalyseur qui, avant son utilisation, a été réduit avec de l'hydrogène à des températures de 20 à 300°C.
